(19) Europäisches Patentamt

European Patent Office

Office européen des brevets

(11) Publication number : **0 331 948 B1**

# (12) EUROPEAN PATENT SPECIFICATION

(45) Date of publication of patent specification :
**15.01.92 Bulletin 92/03**

(51) Int. Cl.⁵ : **A61K 31/725,** A61K 47/00, A61K 9/06

(21) Application number : **89102823.5**

(22) Date of filing : **18.02.89**

(54) Pharmaceutical composition for the transdermal administration of heparin.

(30) Priority : **22.02.88 DE 3805523**

(43) Date of publication of application :
**13.09.89 Bulletin 89/37**

(45) Publication of the grant of the patent :
**15.01.92 Bulletin 92/03**

(84) Designated Contracting States :
**AT BE CH DE ES FR GB GR IT LI LU NL SE**

(56) References cited :
**EP-A- 0 206 947**
**EP-A- 0 214 898**
**CHEMICAL ABSTRACTS, Band 108, Nr. 4, 25.**
**Januar 1988, Zusammenfassung Nr. 26969w,**
**Columbus, Ohio, US; & JP-A-62 153 227 (SEKI-**
**SUI CHEMICAL CO., LTD) 08-07-1987**

(73) Proprietor : **SCHUR PHARMAZEUTIKA GMBH**
**& CO KG**
**Emanuel-Leutze-Strasse 1**
**W-4000 Düsseldorf 11 (DE)**
Proprietor : **Stüttgen, G., Prof. Dr. med.**
**Augustenburger Platz 1**
**W-1000 Berlin 65 (DE)**

(72) Inventor : **Stüttgen, G., Prof.Dr.med.**
**Augustenburger Platz 1**
**W-1000 Berlin 65 (DE)**
Inventor : **Gehlhaar, Klaus**
**Rheinallee 106**
**W-4000 Düsseldorf 12 (DE)**

(74) Representative : **Redies, Bernd, Dr. rer. nat.**
**COHAUSZ & FLORACK Patentanwaltsbüro**
**Schumannstrasse 97 Postfach 14 01 20**
**W-4000 Düsseldorf 1 (DE)**

Jouve, 18, rue Saint-Denis, 75001 PARIS

## Description

The present invention is related to pharmaceutical compositions for the transdermal application of heparin.

The skin is a natural barrier for the human body. Many compounds producing their activity within the body, i.e. systemically, either poisonous compounds or drugs, do not overcome or only with difficulties overcome this natural protective barrier and produce their systemical activity only after purposely avoiding this protective barrier, for instance as with many drugs, by administering them perorally or, as with heparin, by intravenous injection, or unobviously avoiding it in that such poisonous materials are taken perorally by chance. The transdermal administration of drugs is possible only in a few cases such as with nitroglycerine. Further difficulties arise in particular with active agents having a polar molecule as they often occur in nature such as with heparin as active agent.

Tests have been run for over 20 years to administer heparin with its high molecular and anionic molecule topically for certain diseases which are mainly caused by thrombotic problems, a shortening of the time of blood clotting and/or fibrinolysis. At present, the results obtained are so disappointing, that up to now there has not been marketed a composition for transdermal application of heparin producing systemically the activity of heparin.

The object of the present invention is to provide a pharmaceutical preparation for the transdermal application of heparin, i.e. avoiding or substantially removing the protective barrier function of the skin.

In the compositions according to the present invention heparin is combined with a salt of choline and a fatty acid having 12 to 20 carbon atoms in the alkyl group of this fatty acid. The combination of heparin and such salt of choline is presented to the skin in a usual ointment base. Preferably the salt of choline with the fatty acid is a salt of a fatty acid having 16 to 18 carbon atoms in the alkyl group and having a saturated chain, in particular stearic acid. Preferably the fatty acid is present in a surplus based upon the amount of the choline base in comparison to the equivalent amount for salt formation.

The ointment base preferably is an oil in water emulsion (O/W-emulsion). Most preferably the ointment base is that of the product CHOMELANUM® of applicant.

Heparin is present preferably in an amount corresponding to 100,000 to 10,000,000 I.U. (international units) per mole of the choline salt, preferably the above most preferred choline stearate. There may be present larger amounts depending upon the amount of heparin compatible with the ointment base. The composition should offer to the skin as much heparin per area as possible in order to produce a heparin "administration pressure" of the ointment composition to the skin as high as possible. The pharmaceutical compositions according to the invention preferably comprise the choline fatty acid salt in an amount of 2 to 10 g per 100 g of the final pharmaceutical composition, preferably 2 to 5 g per 100 g of the final pharmaceutical composition.

It has been found surprisingly that the natural barrier function of the skin as it is found with heparin, substantially is removed and the slow flux of heparin through the skin is increased very considerably if heparin is offered to the skin together with the fatty acid salt of choline in ointments, in particular in the form of oil-in-water emulsions.

The pharmaceutical compositions according to the present invention are produced according to the usual procedures for producing ointments containing active agents and to be applied to the skin, by mixing the components in a mixing apparatus suitable therefor.

Example :

Product "chomelanum ointment" for 100 g of final product :

```
4.425 g of choline stearate 66%
2.250 g of stearic acid
8.500 g of emulsified cetyl stearyl alcohol (product
            Lanette N)
0.025 g of base Bois PH 799997
0.500 g of benzyl alcohol
0.500 g of 2-phenoxyethanol
83.800 g of purified water
        ----------
100.000 g
```

This ointment base is thoroughly mixed with a usual preparation of heparin containing 50,000 I.U. of heparin such that the final ointment preparation contains 50,000 I.U. heparin per 2.92 g of pure choline stearate and an equimolar amount of free additional stearic acid in an oil-in-water emulsion.

## Claims

1. Pharmaceutical composition for the transdermal application of heparin, characterized in that the preparation in addition to the active agent contains a salt of choline and a fatty acid having 12 to 20 carbon atoms in the alkyl group besides usual carrier and addition products of an ointment base.

2. Pharmaceutical composition according to claim 1, characterized in that the choline salt of the fatty acid is a salt of a fatty acid with 16 to 18 carbon atoms in the alkyl group and having a saturated alkyl chain.

3. Pharmaceutical composition according to claim 1 or 2, characterized in that the fatty acid salt of choline is choline stearate.

4. Pharmaceutical composition for the transdermal application of heparin according to claims 1 to 3, characterized in that there is present heparin and the choline fatty acid salt in an ointment based on an oil-in-water emulsion.

5. Pharmaceutical composition according to claim 4, characterized in that it additionally comprises the free fatty acid of said choline fatty acid salt.

## Patentansprüche

1. Pharmazeutische Präparate zur transdermalen Applikation von Heparin, **dadurch gekennzeichnet**, daß die Präparate zusätzlich zu dem Wirkstoff ein Salz des Cholins und einer Fettsäure mit 12 bis 20 Kohlenstoffatomen im Alkylrest neben üblichen Träger- und Zusatzstoffen für eine Salbengrundlage enthält.

2. Pharmazeutische Präparate gemäß Anspruch 1, **dadurch gekennzeichnet**, daß das Cholinsalz der Fettsäure ein solches einer Fettsäure mit 16 bis 18 Kohlenstoffatomen im Alkylrest mit gesättigter Alkyl-Kette ist.

3. Pharmazeutische Präparate gemäß Anspruch 1 oder 2, **dadurch gekennzeichnet**, daß das Cholinfettsäuresalz Cholinstearat ist.

4. Pharmazeutische Präparate zur transdermalen Applikation von Heparin gemäß Ansprüchen 1 bis 3, **dadurch gekennzeichnet**, daß Heparin und Cholinsalz in einer Salbe auf der Grundlage einer Öl-in-Wasser-Emulsion vorliegen.

5. Pharmazeutische Präparate gemäß Anspruch 4, **dadurch gekennzeichnet**, daß die Präparate zusätzlich die freie Fettsäure des Cholinfettsäuresalzes enthalten.

## Revendications

1. Composition pharmaceutique pour l'application percutanée d'héparine, caractérisée en ce que la préparation, en plus de l'agent actif, contient un sel de choline d'un acide gras ayant 12 à 20 atomes de carbone dans le groupe alkyle en plus du véhicule habituel et des produits additionnels d'une base pour pommade.

2. Composition pharmaceutique selon la revendication 1, caractérisée en ce que le sel de choline de l'acide gras est un sel d'un acide gras dont le groupe alkyle a 16 à 18 atomes de carbone et dont la chaîne alkyle est saturée.

3. Composition pharmaceutique selon la revendication 1 ou 2, caractérisée en ce que le sel d'acide gras de choline est le stéarate de choline.

4. Composition pharmaceutique pour l'application percutanée de l'héparine selon les revendications 1 à 3, caractérisée en ce que l'héparine et le sel de choline d'acide gras sont présents dans une pommade à base d'émulsion huile dans l'eau.

5. Composition pharmaceutique selon la revendication 4, caractérisée en ce qu'elle comprend de plus l'acide gras libre dudit sel de choline d'acide gras.